# EUROPÄISCHE PATENTANMELDUNG

(11) **EP 0 798 322 A1**
(43) Veröffentlichungstag der Anmeldung: **01.10.1997**
(21) Anmeldenummer: 97101423.8
(22) Anmeldetag: 30.01.1997
(51) Int. Cl.: C08G 18/02, C07C 267/00

(54) **Carbodiimidgruppen enthaltende blockierte (cyclo)aliphatische Diisocyanate, ein Verfahren zu ihrer Herstellung sowie ihre Verwendung**

(30) Priorität: 26.03.1996 DE 19611820
(71) Anmelder: HÜLS AKTIENGESELLSCHAFT, 45764 Marl (DE)
(72) Erfinder: Gras, Rainer, Dr., 44879 Bochum (DE); Wolf, Elmar, Dr., 45661 Recklinghausen (DE)

(57) **Zusammenfassung**

Blockierte (cyclo)aliphatische Diisocyanate, dadurch gekennzeichnet, daß sie eine Carbodiimidgruppe enthalten und folgender Zusammensetzung entsprechen: 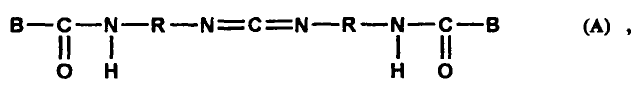 wobei R ein (Cyclo)alkylen-Rest mit 4 - 16 C-Atomen, der mit 1 - 4 CH₃-Gruppen substituiert sein kann, und B Reste von Lactamen, Oximen und/oder Triazolen bedeutet.

## Beschreibung

Gegenstand der vorliegenden Erfindung sind Carbodiimidgruppen enthaltende blockierte (cyclo)aliphatische Diisocyanate, ein Verfahren zu ihrer Herstellung sowie ihre Verwendung.

Während Polycarbodiimide eine seit langem bekannte Verbindungsklasse zur Hydrolysestabilisierung darstellen, sind Carbodiimidgruppen enthaltende (cyclo)aliphatische Diisocyanataddukte nicht bekannt. Dies gilt gleichermaßen auch für die entsprechend blockierten Diisocyanataddukte.

Aufgabe der Erfindung war somit die Bereitstellung dieser Verbindungen.

Überraschenderweise konnten carbodiimidgruppenhaltige blockierte (cyclo)aliphatische Diisocyanate dadurch hergestellt werden, daß man monoblockierte (cyclo)aliphatische Diisocyanate, die weitgehend frei von diblockierten Diisosocyanaten und monomeren Diisosocyanaten sind, katalytisch erhitzt.

Gegenstand der vorliegenden Erfindung sind daher blockierte (cyclo)aliphatische Diisocyanate, dadurch gekennzeichnet, daß sie eine Carbodiimidgruppe enthalten und folgender Zusammensetzung entsprechen: 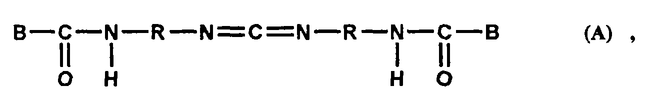 wobei R ein (Cyclo)alkylen-Rest mit 4 - 16 C-Atomen, der mit 1 - 4 CH₃-Gruppen substituiert sein kann, und B Reste von Lactamen, Oximen und/oder Triazolen bedeutet.

Die erfindungsgemäßen Verbindungen enthalten im Durchschnitt 1 mol Carbodiimidgruppen (-N=C=N-) pro mol (A). Der blockierte NCO-Gehalt liegt bei 10 - 20 %. Weiterhin weisen die erfindungsgemäßen Verbindungen einen Schmelzbereich von 70 - 140 °C auf.

Neben ihrem latenten NCO-Gehalt, der zu Vernetzungsreaktionen befähigt, weisen die erfindungsgemäßen Verbindungen zusätzlich einen hydrolysestabilisierenden Effekt auf.

Für die erfindungsgemäßen Carbodiimidgruppen enthaltenden blockierten (cyclo)aliphatischen Diisocyanate kommen alle Diisocyanate in Frage, die sich bei einer Temperatur destillieren lassen, die niedriger ist als die Deblockierungstemperatur des monoblockierten Diisocyanats. Als besonders geeignet haben sich folgende Diisocyanate erwiesen: Hexamethylendiisocyanat (HDI), 2-Methyl-1.5-diiso-cyanatopentan (DI 51), 1.5-Diisocyanatohexan, 1.10-Diisocyanatodecan, 1.9-Diisocyanato-5-methylnonan, Dodecamethylendiisocyanat, Isophorondiisocyanat (IPDI), 2.2.4(2.4.4)-Trimethylhexamethylendiisocyanat (TMDI), Hexahydroxylylendiisocyanat-1.4 oder -1.3. Die Diisocyanate können allein oder auch als Mischungen in den erfindungsgemäßen Verbindungen enthalten sein.

Als Blockierungsmittel werden ε-Caprolactam, Oxime, wie z. B. Acetonoxim, Methylethylketoxim, Acetophenonoxim und Triazole, wie z.B. 1.2.4-Triazol, bevorzugt eingesetzt. Die Blockierungsmittel können allein oder als Mischungen in den erfindungsgemäßen Verbindungen enthalten sein.

Der Gehalt der monoblockierten Diisosocyanate, die nicht Gegenstand der Erfindung sind, sondern nur die Ausgangsverbindungen für die erfindungsgemäßen Verbindungen darstellen, beträgt - je nach Diisocyanat - 11 - 18 Gew.-%, an Gesamt-NCO 23 - 35 Gew.-%. Das Molverhältnis von Diisocyanat zu Blockierungsmittel hängt davon ab, welcher Gehalt an diblockiertem Diisocyanat zugelassen werden kann. Je höher der Überschuß an Diisocyanat, desto niedriger ist der Gehalt an diblockiertem Diisocyanat.

Ein weiterer Gegenstand der vorliegenden Erfindung ist ein Verfahren zu ihrer Herstellung, dadurch gekennzeichnet, daß man in einer 1. Stufe 5 - 20 mol Diisocyanat und 1 mol Blockierungsmittel (B-H) bei 60 - 120 °C umsetzt, und nach erfolgter Umsetzung das nicht umgesetzte Diisocyanat in einer 2. Stufe bei 90 - 140 °C/0,1 mbar abtrennt, und anschließend das so erhaltene Zwischenprodukt bei 120-180 °C mit 0,1 - 3 Gew.-% eines Carbodiimidisierungskatalysators so lange erhitzt, bis der NCO-Gehalt des Reaktionsprodukts (A) ≥ 0,1 % beträgt.

Bei der Umsetzung von überschüssigem Diisocyanat und Blockierungsmittel geht man so vor, daß man zu 5 - 20 Molen Diisocyanat bei 60 - 120 ° C 1 Mol Blockierungsmittel zudosiert und so lange weitererhitzt, bis ein 1 NCO-Äquivalent verbraucht ist. Der Reaktionsverlauf wird durch Bestimmung des NCO-Gehaltes verfolgt. Ist der erwartete NCO-Gehalt erreicht, wird das Reaktionsprodukt in einer 2. Stufe vom nicht umgesetzten Diisocyanat z. B. durch eine Dünnschichtdestillation bei ca. 0,1 - 0,2 mbar entfernt. Nach dem erfindungsgemäßen Verfahren wird nun das Reaktionsprodukt der 2. Stufe bei 120 - 180 °C mit 0,1 - 3 Gew.-% eines Carbodiimidisierungskatalysators, wie z. B. Tributylphosphinoxid, oder Phospholinoxide, wie z. B. 1-Methyl-1-oxophospholin, 1-Ethyl-1-oxophospholin, 1-Phenyl-1-oxophospholin, 1.3-Dimethyl-1-oxophospholin, 1.3.4-Triinethyl-1-oxophospholin, so lange unter intensivem Rühren erhitzt, bis der NCO-Gehalt des Reaktionsprodukts (A) ≥ 0,1 % beträgt.

Ein weiterer Gegenstand der vorliegenden Erfindung betrifft die Verwendung der erfindungsgemäßen Verbindungen in Kombination mit OH-Gruppen enthaltenden Polymeren als Bindemittel für lichtbeständige Polyurethan-Pulverbeschichtungen.

Die hitzehärtbare pulverförmige Masse, in der das erfindungsgemäße Carbodiimidgruppen enthaltende blockierte (cyclo)aliphatische Diisocyanat eingesetzt wird, enthält:
a) 100 Gew.-T. OH-gruppenhaltige Polymere
b) 15 - 60 Gew.-T. blockiertes Diisocyanat (A)
c) 0 - 160 Gew.-T. Pigmente
d) 0 - 200 Gew.-T. übliche Füllstoffe
e) 0 - 5 Gew.-T. Katalysator
f) 0,5 - 5 Gew.-T. Verlaufsmittel

Der Bestandteil a kann grundsätzlich jedes mehr als zwei OH-Gruppen enthaltende Polymer sein, das bei mindestens 70 °C schmilzt. Hierbei handelt es sich um Polyetherpolyole, Polyesteramidpolyole, Polyurethanpolyole, hydroxylierte Acrylharze usw., deren OH-Gruppen für die Vernetzung mit den erfindungsgemäßen Carbodiimidgruppen enthaltenden blockierten (cyclo)aliphatischen Diisocyanaten bestimmt sind, Besonders bevorzugt sind unter den zahlreichen Möglichkeiten für hydroxylgruppentragende Polymere im Rahmen der Erfindung Polyesterpolyole. Solche Polyesterpolyole sollen ein Molgewicht zwischen 1000 - 3000, vorzugsweise zwischen 1500 und 2500, und eine OH-Zahl von 30 - 240 haben. Solche Polyesterpolyole werden z. B. in den DE-OS 19 57 483, 25 42 191, 30 04 876 und
31 43 060 beschrieben.

Um die Geliergeschwindigkeit der hitzehärtbaren Pulverlacke zu erhöhen, kann man Katalysatoren zusetzen. Als Katalysatoren verwendet man Organozinnverbindungen wie Dibutylzinndilaurat (DBTL), Sn(II)-octoat, Dibutylzinnmaleat usw.. Die Menge an zugesetztem Katalysator beträgt 0,1 - 5 Gew.-T. auf 100 Gew.-T. des Hydroxylgruppen tragenden Polyesters.

Die Isocyanatkomponente wird für die Herstellung von PUR-Pulverlacken mit dem geeigneten hydroxylgruppenhaltigen Polymeren und gegebenenfalls Katalysatoren sowie Pigmenten und üblichen Hilfsmitteln wie Füllstoffen und Verlaufsmitteln, z. B. Siliconöl, Acrylatharzen, gemischt und in der Schmelze homogenisiert. Dies kann in geeigneten Aggregaten, wie z. B. beheizbaren Knetern, vorzugsweise jedoch durch Extrudieren, erfolgen, wobei Temperaturobergrenzen von 130 - 140 °C nicht überschritten werden sollten. Die extrudierte Masse wird nach Abkühlen auf Raumtemperatur und nach geeigneter Zerkleinerung zum sprühfertigen Pulver vermahlen. Das Auftragen des sprühfertigen Pulvers auf geeignete Substrate kann nach den bekannten Verfahren, wie z. B. durch elektrostatisches Pulversprühen, Wirbelsintern, elektrostatisches Wirbelsintern, erfolgen. Nach dem Pulverauftrag werden die beschichteten Werkstücke zur Aushärtung 60 bis 4 Minuten auf eine Temperatur von 150 - 220 °C, vorzugsweise 30 bis 6 Minuten bei 160 - 200 °C, erhitzt.

Die erfindungsgemäßen PUR-Pulverlacke zeichnen sich durch hervorragende Witterungs- und sehr gute Farbbeständigkeit aus.

### Experimenteller Teil

### A) Herstellung der erfindungsgemäßen Verbindungen

10 mol Diisocyanat und 1 mol Blockierungsmittel (B-H) werden bei 60 - 120 °C in gewohnter Weise (portionsweise Zudosierung des Blockierungsmittels) umgesetzt und nach erfolgter Umsetzung das nicht umgesetzte Diisocyanat in einer 2. Stufe bei 90 - 140 °C/0,1 mbar abgetrennt, und anschließend das so erhaltene Zwischenprodukt bei 120 - 180 °C mit 0,3 Gew.-% 1-Methyl-1-oxophospholin so lange erhitzt (ca. 3 h), bis der NCO-Gehalt des Reaktionsproduktes ≤ 0,1 Gew.-% beträgt. Die in der Tabelle 1 angegebenen Verbindungen wurden nach diesem Verfahren hergestellt.

**Tabelle 1**

| **Zusammensetzung der erfindungsgemäßen, eine Carbodiimidgruppe enthaltenden Verbindungen** | | | | | |
|---|---|---|---|---|---|
| **Beispiel A** | **Zusammensetzung in mol** | | **NCO- frei [%]** | **NCO- blockiert [%]** | **Smp. [°C]** |
| | **Diisocyanat** | **Blockierungsmittel** | | | |
| 1 | 2 IPDI | 2 ε-Caprolactam | 0,1 | 13,66 | 113 - 120 |
| 2 | 2 IPDI | 2 Acetophenonoxim | 0,1 | 12,62 | 125 - 132 |
| 3 | 2 HDI | 2 ε-Caprolactam | 0,1 | 16,35 | 84 - 101 |
| 4 | 2 TMDI | 2 ε-Caprolactam | 0,1 | 14,10 | 86 - 95 |
| 5 | 2 DI 51 | 2 1.2.4-Triazol | 0,1 | 18,60 | 95 - 109 |

### B Polyester

Die folgende Tabelle 2 enthält Beispiele über die zur Formulierung von PUR-Pulverlacken am Markt erhältlichen Polyester.

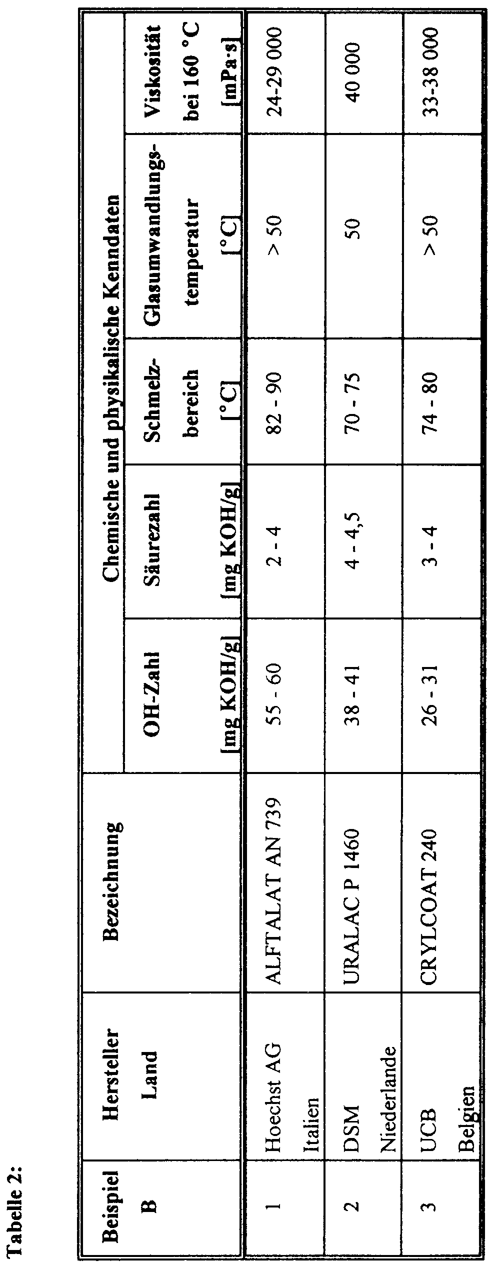

### C Polyurethan-Pulverlacke

### Allgemeine Herstellungsvorschrift

Die zerkleinerten Produkte - blockierte Polyisocyanate (Vernetzer), Polyester, Verlaufsmittel-, ggf. Katalysator-Masterbatch - werden ggf. mit dem Weißpigment in einem Kollergang innig vermischt und anschließend im Extruder bis maximal 130 °C homogenisiert. Nach dem Erkalten wird das Extrudat gebrochen und mit einer Stiftsmühle auf eine Korngröße < 100 µm gemahlen. Das so hergestellte Pulver wird mit einer elektrostatischen Pulverspritzanlage bei 60 KV auf entfettete, ggf. vorbehandelte Eisenbleche appliziert und in einem Umlufttrockenschrank bei Temperaturen zwischen 170 und 200 °C eingebrannt.

### Verlaufsmittel-Masterbatch

Es werden 10 Gew.-% des Verlaufsmittels - ein handelsübliches Copolymer von Butylacrylat und 2-Ethylhexylacrylat - in dem entsprechenden Polyester in der Schmelze homogenisiert und nach dem Erstarren zerkleinert.

### Katalysator-Masterbatch

Es werden 5 Gew.-% des Katalysators - DBTL - in dem entsprechenden Polyester in der Schmelze homogenisiert und nach dem Erstarren zerkleinert.

Die Abkürzungen in den folgenden Tabellen bedeuten:
- S.D.: = Schichtdicke in µm
- ET: = Tiefung nach Erichsen in mm (DIN 53 156)
- GS: = Gitterschnittprüfung (DIN 53 151)
- GG 60 °∢: = Messung des Glanzes nach Gardner (ASTM-D 5233)
- Imp. rev.: = Impact reverse in g·m
- HK: = Härte nach König in sec (DIN 53 157)

### C 1 Pigmentierte Pulverlacke

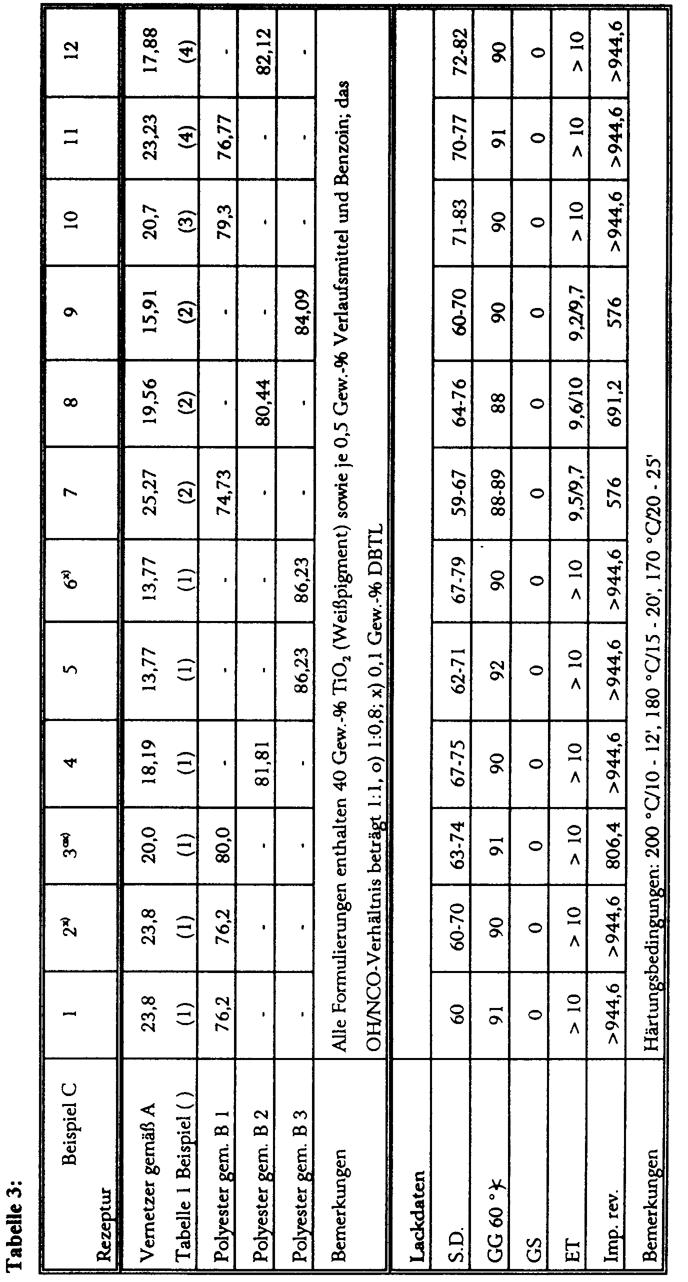

### C2 Transparente Pulverlacke

Nach dem beschriebenen Verfahren wurden auch die transparenten Pulverlacke hergestellt, appliziert und zwischen 200 und 160 °C eingebrannt.

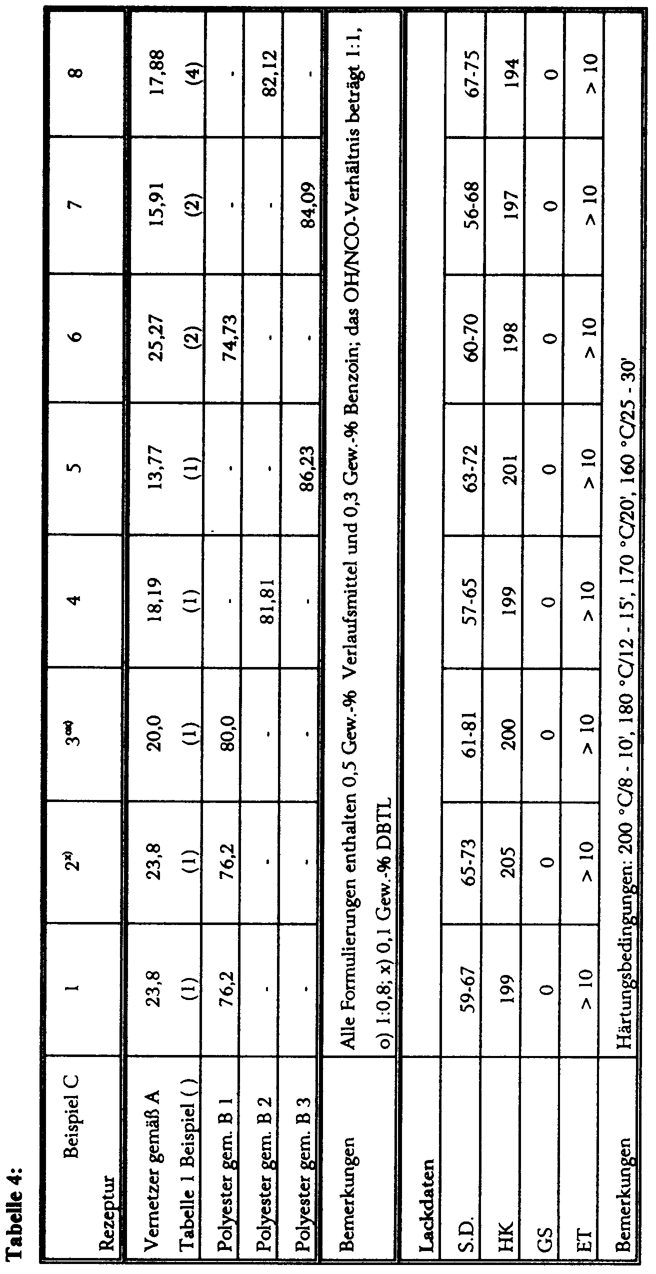

## Patentansprüche

1. Blockierte (cyclo)aliphatische Diisocyanate,
dadurch gekennzeichnet,
daß sie eine Carbodiimidgruppe enthalten und folgender Zusammensetzung entsprechen: 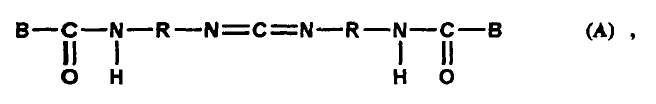 wobei R ein (Cyclo)alkylen-Rest mit 4 - 16 C-Atomen, der mit 1 - 4 CH₃-Gruppen substituiert sein kann, und B Reste von Lactamen, Oximen und/oder Triazolen bedeutet.

2. Blockierte (cyclo)aliphatische Diisocyanate nach Anspruch 1,
dadurch gekennzeichnet,
daß R Reste Von Hexamethylendiisocyanat (HDI), 2-Methyl-1.5-diisocyanatopentan (DI 51), 1.5-Diisocyanatohexan, 1.10-Diisocyanatodecan, 1.9-Diisocyanato-5-methylnonan, Dodecamethylendiisocyanat, Isophorondiisocyanat (IPDI), 2.2.4(2.4.4)-Trimethylhexamethylendiisocyanat (TMDI), Hexahydroxylylendiisocyanat-1.4 oder -1.3, und beliebige Mischungen dieser Reste bedeutet.

3. Blockierte (cyclo)aliphatische Diisocyanate nach den Ansprüchen 1 oder 2,
dadurch gekennzeichnet,
daß B gleiche oder verschiedene Reste ausgewählt aus 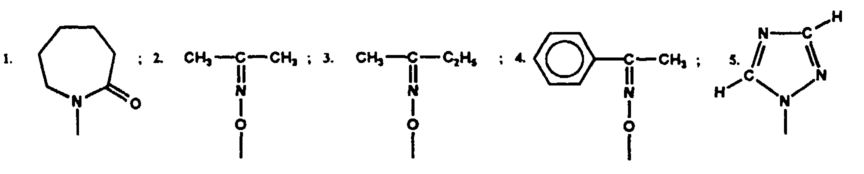 bedeutet.

4. Verfahren zur Herstellung der Verbindungen gemäß den Ansprüchen 1 bis 3,
dadurch gekennzeichnet,
daß man in einer 1. Stufe 5 - 20 mol Diisocyanat (OCN-R-NCO) und 1 mol Blockierungsmittel (B-H) bei 60-120°C umsetzt und nach erfolgter Umsetzung das nicht umgesetzte Diisocyanat in einer 2. Stufe bei 90 - 140 °C/0,1 mbar abtrennt, und anschließend das so erhaltene Zwischenprodukt bei 120 - 180 °C mit 0,1 - 3 Gew.-% eines Carbodiimidisierungskatalysators so lange erhitzt, bis der NCO-Gehalt des Reaktionsprodukts A ≤ 0,1 Gew.-% beträgt.

5. Verfahren nach Anspruch 4,
dadurch gekennzeichnet,
daß als Katalysator Tributylphosphinoxid oder Phospholinoxide, wie z. B. 1-Methyl-1-oxophospholin, 1-Ethyl-1-oxophospholin, 1-Phenyl-1-oxophospholin, 1.3-Dimethyl-1-oxophospholin, 1.3.4-Trimethyl-1-oxophospholin, eingesetzt werden.

6. Verfahren nach den Ansprüchen 4 bis 5,
dadurch gekennzeichnet,
daß das nicht umgesetzte Diisocyanat der 2. Stufe durch eine Dünnschichtdestillation bei reduziertem Druck abgetrennt wird.

7. Verwendung der erfindungsgemäßen Verbindungen nach den Ansprüchen 1 bis 3 in Kombination mit OH-Gruppen enthaltenden Polymeren und weiteren Zusatzstoffen als Bindemittel für lichtbeständige Polyurethan-Pulverbeschichtungen.
